# EUROPÄISCHE PATENTSCHRIFT

(11) **EP 2 134 385 B1**
(45) Veröffentlichungstag und Bekanntmachung des Hinweises auf die Patenterteilung: **11.05.2011**
(21) Anmeldenummer: 08717900.8
(22) Anmeldetag: 17.03.2008
(51) Int. Cl.: A61M 1/10, A61M 1/12

(54) **VORRICHTUNG ZUR UNTERSTÜTZUNG DES HERZENS UND DES KREISLAUFS**
DEVICE FOR SUPPORTING THE HEART AND CIRCULATORY SYSTEM
DISPOSITIF D'ASSISTANCE CARDIAQUE ET CIRCULATOIRE

(30) Priorität: 16.03.2007 DE 102007012817
(43) Veröffentlichungstag der Anmeldung: 23.12.2009
(73) Patentinhaber: Mwf Consult Ltd., 07747 Jena (DE)
(72) Erfinder: FERRARI, Markus, 07747 Jena (DE)
(74) Vertreter: Peckmann, Ralf
(86) Internationale Anmeldenummer: PCT/EP2008/053164
(87) Internationale Veröffentlichungsnummer: WO 2008/113785

(56) Entgegenhaltungen:
- WO-A-2004/078234
- US-A- 4 245 622
- US-A- 4 906 229
- US-B1- 6 607 368

## Beschreibung

Die vorliegende Erfindung betrifft eine Vorrichtung zur Unterstützung des Herzens und des Kreislaufes.

Patienten mit akuter Herzschwäche leiden an einer Minderperfusion lebenswichtiger Organe. Zugleich führt das verminderte Angebot an Sauerstoff-reichem Blut im Kreislauf zu einer Verstärkung der Herzschwäche, da das Herz selber ebenfalls schlechter perfundiert wird. Somit ist bei akut auftretender Herzinsuffizienz eine Unterstützung des Herzens und des Kreislaufes notwendig.

Es ist bekannt, bestimmte Medikamente zur Steigerung der Herzaktion zu verwenden. Da Medikamente die Herzaktion zwar kurzfristig, jedoch auf Kosten des Herzstoffwechsels steigern können, muss bei akutem Herzversagen oder akuter Herzschwäche der Kreislauf mit Hilfe von außen zugeführter Energie in Gang gehalten werden.

Mechanische Kreislaufunterstützungssysteme haben sich in diesem Bereich bewährt, indem sie entweder die Herzaktion unterstützen (intraaortale Ballongegenpulsation, kurz IABP) oder das Kreislaufvolumen durch eigene Pumpaktion erhöhen (axiale Schraubenpumpen, extrakorporale Zirkulationssysteme mit Zentrifugalpumpe). Limitierend beim Einsatz solcher Systeme sind zum einen die Kosten und zum anderen die Möglichkeiten, solche Systeme minimalinvasiv einzubringen. Da diese Pumpsysteme in der Regel über die Leistengefäße mit dem Patientenkreislauf verbunden werden, sind hier Leistungsgrenzen aufgrund der Gefäßinnendurchmesser vorgegeben.

Ein dem Anmelder bekanntes System stellt die intraaortale Ballongegenpulsation (IABP) dar. Dabei wird ein mit Helium-Gas gefüllter Ballonschlauch mit einem Katheter von der Leistenschlagader bis zur thorakalen Aorta descendens vorgebracht und hier gegenläufig zur Herzaktion abrupt aufgeblasen bzw. wieder evakuiert. Durch die Tätigkeit der IABP wird die Nachlast für die linke Herzkammer gesenkt, woraus eine Senkung des myokardialen Sauerstoffverbrauches resultiert. Durch das abrupte Aufblasen des Ballons in der Diastole kommt es zu einem Blutdruckanstieg und damit besserer Perfusion von Organen und Herz.

Nachteilig an diesem Ansatz hat sich die Tatsache herausgestellt, dass die IABP nur bei ausreichender Eigenleistung des Herzens eine suffiziente Augmentation bewirkt. Im Falle eines Herzstillstandes ist eine IABP funktionell wirkungslos. Eine IABP-Einheit ist bei Patienten, die eine höhergradige Aortenklappeninsuffizienz aufweisen, kontraindiziert, da es infolge des Aufblasens des IABP-Ballons zum Überladen des linken Ventrikels kommt.

Die US 4,906,229 beschreibt eine Vorrichtung zum Pumpen von Blut in einem Lebewesen von einem ersten Ort zu einem zweiten Ort. Die Vorrichtung weist ein starres zylinderförmiges Gehäuse auf, an dessen Innenwand anliegend eine flexible Membran angeordnet ist. Durch Einbringen eines Steuerfluides zwischen die Innenwand und die flexible Membran ist diese von der Innenwandung des Gehäuses abhebbar bzw. zu dieser hin bewegbar. Hierdurch wird ein Pumpvorgang ermöglicht. Um die Richtung des Blutstromes durch das Gehäuse zu steuern, sind jeweils an Stirnseiten des Gehäuses gleichsinnig orientierte Klappenventile angeordnet. Das Gehäuse ist fluiddicht mit einer Kanüle verbunden, deren dem Gehäuse abgewandtes Ende in eine Herzkammer einführbar ist. Die Vorrichtung ermöglicht so ein Pumpen von Blut von dem ersten Ort zu dem zweiten Ort.

Es ist Aufgabe der vorliegenden Erfindung, eine Vorrichtung zur Unterstützung des Herzens und des Kreislaufs zu schaffen, welche die oben genannten Nachteile beseitigt.

Erfindungsgemäß wird eine Vorrichtung zur Zirkulation einer Körperflüssigkeit in einem Körper eines Lebewesens, insbesondere zum Unterstützen des Herzens und/oder des Kreislaufes des Lebewesens, gemäß Anspruch 1 vorgeschlagen, welche eine Kathetereinrichtung, welche mindestens einen Einlassabschnitt zur Aufnahme der Körperflüssigkeit an mindestens einem ersten Ort innerhalb des Körpers des Lebewesens, mindestens einen von dem mindestens einen Einlassabschnitt beabstandeten Auslassabschnitt zur Abgabe der Körperflüssigkeit an mindestens einem von dem mindestens einen ersten Ort beabstandeten zweiten Ort innerhalb des Körpers des Lebewesens und eine Pumpeneinrichtung für einen gerichteten Transport der Körperflüssigkeit zwischen dem mindestens einen Einlassabschnitt und dem mindestens einen Auslassabschnitt der Kathetereinrichtung aufweist; und eine mit der Kathetereinrichtung gekoppelte Ventilanordnung aufweist für eine in Abhängigkeit des Betriebes der Pumpeneinrichtung gesteuerte Aufnahme der Körperflüssigkeit an dem mindestens einen Einlassabschnitt der Kathetereinrichtung und gesteuerte Abgabe der Körperflüssigkeit an dem mindestens einen Auslassabschnitt der Kathetereinrichtung.

Die vorliegende Erfindung weist demnach gegenüber dem zitierten Stand der Technik den Vorteil auf, dass bei wenig Fremdoberfläche und geringem Diameter am Herzen eine große Pumpleistung gewährleistet wird. Ferner besteht die Möglichkeit einer Herzentlastung und Kreislaufstabilisierung in abgestufter bzw. zur Herzaktion synchronisierter Weise. Da durch die Pumpenanordnung Blut aktiv aus beispielsweise dem linken Ventrikel herausgesaugt werden kann, kann die Vorrichtung auch bei Patienten eingesetzt werden, die eine höhergradige Aortenklappeninsuffizienz aufweisen.

Durch leichte Modifikation der Vorrichtung kann diese vorteilhaft entweder im Bereich der linken Herzkammer oder im Bereich der rechten Herzkammer je nach Bedarf eingesetzt werden.

In den Unteransprüchen werden vorteilhafte Ausgestaltungen der erfindungsgemäßen Vorrichtung näher erläutert.

Im Folgenden werden bevorzugte Ausführungsbeispiele der Erfindung anhand der Figuren der Zeichnung näher erläutert.

Von den Figuren zeigen:
- Fig. 1a: eine schematische Darstellung einer Vorrichtung zur Herz-/Kreislaufunterstützung gemäß einem bevorzugten Ausführungsbeispiel der vorliegenden Erfindung in einem ersten Zustand;
- Fig. 1b: eine schematische Darstellung der Vorrichtung aus Fig. 1a in einem zweiten Zustand;
- Fig. 2: eine schematische Darstellung einer Vorrichtung zur Herz-/Kreislaufunterstützung gemäß einem weiteren be- vorzugten Ausführungsbeispiel der vorliegenden Erfin- dung, wobei die Vorrichtung zur Unterstützung der lin- ken Herzkammer ausgebildet und eingesetzt ist;
- Fig. 3: eine schematische Darstellung einer Vorrichtung zur Herz-/Kreislaufunterstützung gemäß einem weiteren be- vorzugten Ausführungsbeispiel der vorliegenden Erfin- dung, wobei die Vorrichtung zur Unterstützung der lin- ken Herzkammer ausgebildet und eingesetzt ist; und
- Fig. 4: eine schematische Darstellung einer Vorrichtung zur Herz-/Kreislaufunterstützung gemäß einem weiteren be- vorzugten Ausführungsbeispiel der vorliegenden Erfin- dung, wobei die Vorrichtung zur Unterstützung der rech- ten Herzkammer ausgebildet und eingesetzt ist.

In den Figuren bezeichnen gleiche Bezugszeichen gleiche oder funktionsgleiche Komponenten, soweit nichts Gegenteiliges angegeben ist.

An dieser Stelle sei darauf hingewiesen, dass in den Figuren Flüssigkeitsbewegungen bzw. Strömungsrichtungen durch Pfeile gekennzeichnet sind.

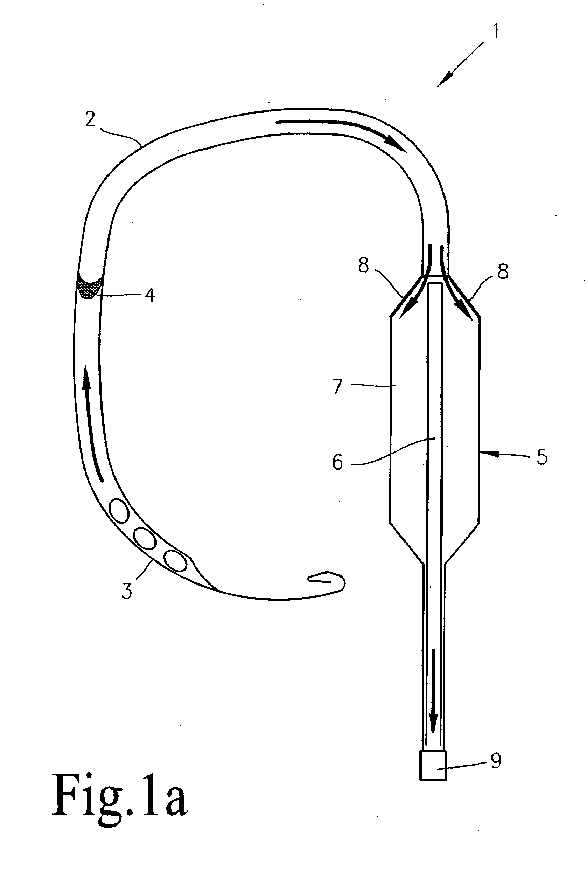

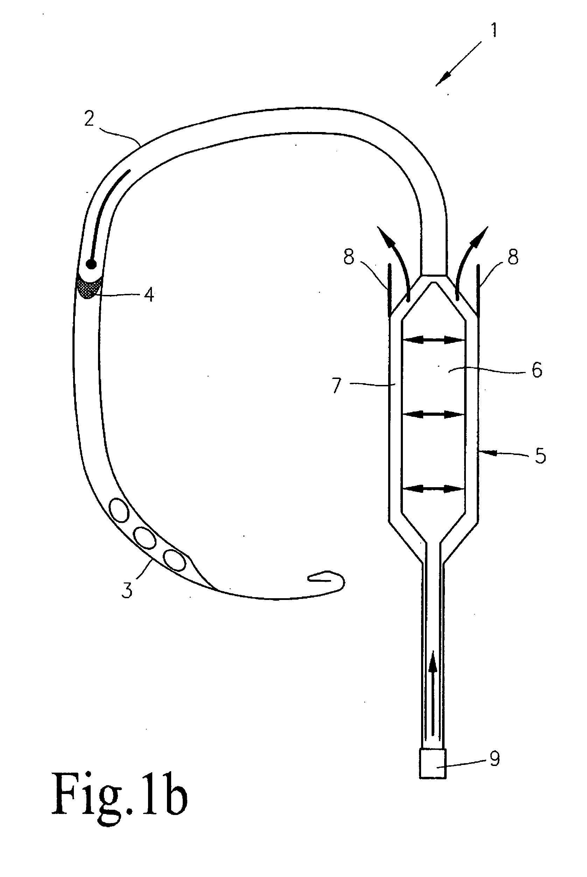

In den Fig. 1a und 1b ist jeweils eine Vorrichtung 1 zur Unterstützung des Herzens und/oder des Kreislaufes eines Lebewesens gemäß einem bevorzugten Ausführungsbeispiel der vorliegenden Erfindung illustriert. Dabei zeigt Fig. 1a die Vorrichtung 1 in einem ersten Zustand und Fig. 1b die Vorrichtung 1 in einem zweiten Zustand.

Wie in den Fig. 1a und 1b ersichtlich ist, weist die Vorrichtung 1 eine Pumpenkanüle 2 auf, welche einem Transport der Körperflüssigkeiten von einem ersten Ort an einen zweiten Ort im Inneren des Körpers des Lebewesens dient. An dem einen Ende der Pumpenkanüle 2 ist ein Kanülenende 3 mit Öffnungsbereichen angeordnet. Das Kanülenende 3 dient beim vorliegenden Ausführungsbeispiel beispielsweise als Einlassabschnitt zur Aufnahme der Körperflüssigkeit an dem ersten Ort innerhalb des Körpers des Lebewesens. Gemäß dem vorliegenden Ausführungsbeispiel kann das Kanülenende 3 auch als Ansaugkanüle 3 bezeichnet werden.

Ferner ist in den Fig. 1a und 1b ersichtlich, dass benachbart zu dem Kanülenende 3 innerhalb der Pumpenkanüle 2 ein Einlassventil 4 angeordnet ist, welches im geöffneten Zustand nach Fig. 1a die Körperflüssigkeit von dem Kanülenende 3 durchlässt und im geschlossenen Zustand nach Fig. 1b die Körperflüssigkeit nicht mehr zu dem Kanülenende 3 zurückströmen lässt.

Im Bereich des dem Kanülenende 3 gegenüberliegenden Endes der Vorrichtung 1 ist eine Pumpeneinrichtung 5 integriert, welche beispielsweise aus einem zusammenfaltbaren Doppelkammer-Hohlkörper mit einer inneren Kammer 6 und einer äußeren Kammer 7 besteht. Ferner umfasst die Pumpeneinrichtung 5 mehrere, beispielsweise zwei Auslassventile 8. Die Aulassventile 8 sind beispielsweise in der äußeren Wandung der äußeren Kammer 7 der Pumpeneinrichtung 5 in einem Auslassabschnitt der Vorrichtung integriert, welcher benachbart zu der Pumpenkanüle 2 vorgesehen ist. Es ist allerdings für einen Fachmann offensichtlich, dass die Einlass- und Auslassventile in ihrer Anordnung, Anzahl und Ausgestaltung entsprechend der jeweiligen Verwendung modifiziert und optimiert ausgelegt werden können.

Zudem ist vorzugsweise mit der Pumpeneinrichtung 5 eine Antriebseinrichtung 9 gekoppelt, welche in den Fig. 1a und 1b schematisch mit dem Bezugszeichen 9 gekennzeichnet ist. Die Antriebseinrichtung kann beispielsweise als externe oder als in dem Körper des Lebewesens in miniaturisierter Weise integrierte Antriebseinrichtung oder auf andere vorteilhafte Weise ausgebildet sein.

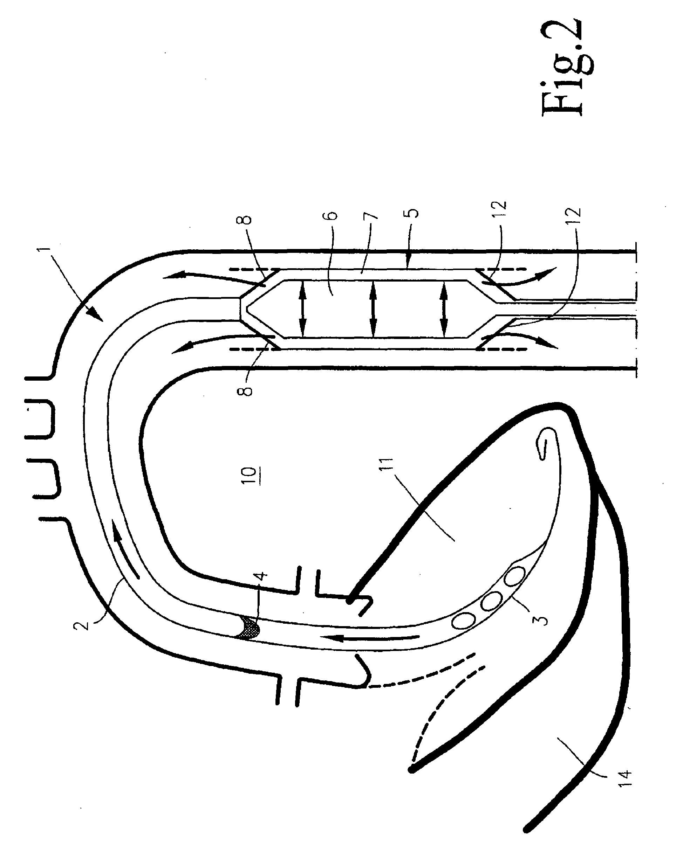

Fig. 2 illustriert eine schematische Darstellung einer Vorrichtung 1 gemäß einem weiteren bevorzugten Ausführungsbeispiel. In Fig. 2 ist die Vorrichtung 1 beispielhaft im linken Ventrikel bzw. in der linken Herzkammer 11 zur Unterstützung derselben eingebracht. Bei einem Öffnen des Einlassventils 4 wird Blut von dem linken Ventrikel 11 über die Pumpenkanüle 2 in die äußere Kammer 7 der Pumpeneinrichtung 5 gesogen, wie in Fig. 1a schematisch dargestellt ist. Bei Aufblasen der inneren Kammer 6 der Pumpeneinrichtung 5, beispielweise als Pumpballon ausgebildet, wird das Blut anschließend mittels der Auslassventile 8 in den Kreislauf abgegeben (siehe die schematische Darstellung in Fig. 1b, Fig. 2 und Fig. 3).

Das Kanülenende 3 kann beispielsweise gerade oder mit einem Pigtail-Ende ausgebildet sein. Der gesamte Katheter wird beispielsweise über die Leistengefäße in den Aorten bogen vorgebracht, wobei die Pumpeneinrichtung in der Aorta descendens liegt.

Im Unterschied zur Vorrichtung gemäß dem ersten Ausführungsbeispiel gemäß den Fig. 1a und 1b weist die Vorrichtung 1 gemäß dem vorliegenden Ausführungsbeispiel nach Fig. 2 zusätzlich zu den beiden oberen Auslassventilen 8 zwei untere Auslassventile 12 in der äußeren Wandung der äußeren Pumpenkammer 7 auf.

Die Funktionsweise der Vorrichtung 1 wird weiter unten detaillierter beschrieben.

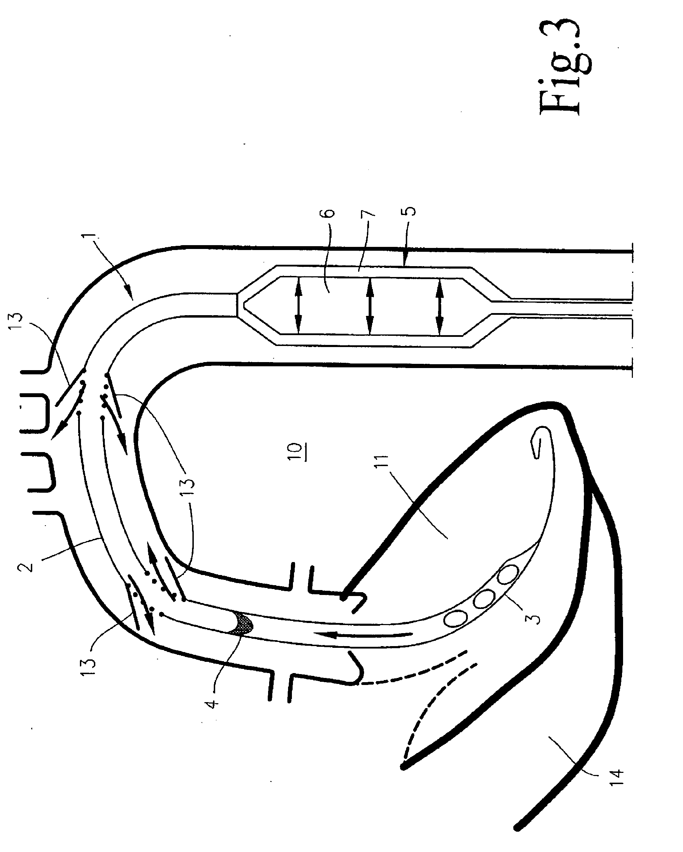

Fig. 3 illustriert eine Vorrichtung 1 gemäß einem weiteren bevorzugten Ausführungsbeispiel, welche ebenfalls zur Unterstützung der linken Herzkammer 11 entsprechend ausgebildet und eingebracht ist. Im Unterschied zum Ausführungsbeispiel gemäß Fig. 2 weist die Vorrichtung 1 keine Auslassventile im Bereich der Pumpeneinrichtung 5 auf. Auslassventile 13 sind im Bereich der Wandung der Pumpenkanüle 2 zwischen der Pumpeneinrichtung 5 und dem Einlassventil 4 vorgesehen. Es ist für einen Fachmann jedoch offensichtlich, dass die Anzahl und Anordnung der Auslassventile 12 entsprechend variiert werden kann und dass eine Kombination der Ausführungsbeispiele gemäß den Figuren 2 und 3 ebenfalls vorstellbar ist.

Die erfindungsgemäße Vorrichtung zur Unterstützung des Herzens und des Kreislaufs gemäß Fig. 1 bis 3 besteht aus einer in der linken Herzkammer 11 zu liegen kommenden Ansaugkanüle 3, welche durch entsprechende Ausarbeitungen einen optimalen Blutflusseinlass in die Pumpenkanüle 2 gewährleistet. Durch ein Einlassventil 4 wird ein gerichteter Blutstrom durch die Pumpenkanüle 2 sichergestellt. Die antreibende Pumpeneinrichtung 5 am Ende der Pumpenkanüle 2 besteht aus einem zusammenfaltbaren Doppelkammer-Hohlkörper, welcher durch mindestens eine fluidgefüllte Pumpenkammer 6 zu einer Volumenverschiebung innerhalb des Pumpsystems führt. Über Auslassventile 8, 12, 13 an der Pumpenkanüle 2 bzw. an der Pumpeneinrichtung 5 wird das Blut während des Aufblasens der Pumpenkammer 6 mit Fluidum herausgepresst. Die Auslassventile 8, 12, 13 sorgen für einen gerichteten Ausstrom der Körperflüssigkeit und verhindern einen Wiedereinstrom in der Füllungsphase. So kann ein gerichteter Fluss innerhalb des Körpers sichergestellt werden, der neben der Entlastung des Herzens eine Verbesserung des Kreislaufvolumens und daraus resultierend der Organperfusion bewirkt.

Da der vorgestellte Pumpenkatheter 1 das Blut aus dem linken Ventrikel heraus saugt (= aktive Entlastung) kann er auch bei Patienten betrieben werden, die eine höhergradige Aortenklappeninsuffizienz (eigentlich Kontraindikation für IABP) aufweisen.

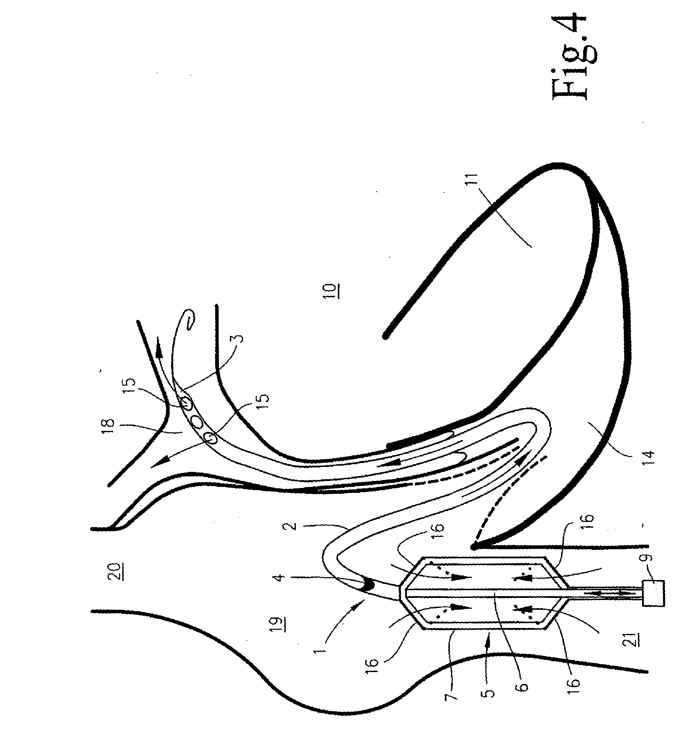

In Fig. 4 ist eine Vorrichtung 1 zur Unterstützung der rechten Herzkammer 14 dargestellt. Die Vorrichtung 1 gemäß dem Ausführungsbeispiel nach Fig. 4 ist derart ausgebildet, dass die Pumpeneinrichtung 5 mehrere Einlassventile 16 aufweist, die analog zu den Auslassventilen 8 gemäß dem Ausführungsbeispiel nach Fig. 2 im Bereich der äußeren Wandung der äußeren Kammer 7 der Pumpeneinrichtung 5 angeordnet sind. Ferner ist das Einlassventil 4 im Inneren der Pumpenkanüle 2 benachbart zu der Pumpeneinrichtung 5 angeordnet. Das der Pumpeneinrichtung gegenüberliegende Ende, d.h. das Kanülenende 3 der Vorrichtung 1 weist vorzugsweise mehrere Auslassventile bzw. Auslassöffnungen 15 auf, so dass die Körperflüssigkeit von der Pumpeneinrichtung 5 über die Pumpenkanüle 2 zu dem Kanülenende 3 transportierbar und über die Aulassventile bzw. Öffnungen 15 an dieser Stelle an das Kreislaufsystem des Lebewesens abgebbar ist.

Fig. 4 zeigt somit ein Ausführungsbeispiel für eine Variante der Vorrichtung für eine Unterstützung der rechten Herzkammer 14. Folglich kann ein Abschnitt der Vorrichtung auch in die A. pulmonalis 18, den rechten Ventrikel 14 entlastend, eingebracht werden. Dabei liegt der Einlassabschnitt der Vorrichtung 1 im rechten Vorhof 19, dem rechten Ventrikel 14 oder der V. cava, insbesondere bei jugulärem Einsatz in der Vena cava superior 20 und bei femoraler Implantation in der vena cava inferior 21.

Durch eine Modifikation der Vorrichtung nach Fig. 1 bis 3, kann die Pumpeneinrichtung 5 auch im Bereich der rechten Herzkammer 14 eingesetzt werden, wie in Fig. 4 dargestellt. In diesem Falle ist die Ansaugkanüle bzw. das Kanülenende 3 im Bereich der Pulmonalarterie endend. Die Ventile 16 im Bereich der Pumpeneinrichtung 5 auf Höhe des rechten Vorhofs oder im Bereich der Hohlvene sorgen für einen Bluteinstrom in dem Moment, in welchem die innere Pumpenkammer 6 ohne Fluidum ist. Bei Anfüllen der inneren Pumpenkammer 6 mit Fluidum schließen die Einlassventile an der äußeren Pumpenkammer 7 und das Blut wird durch den Pumpenkatheter 2 hindurch zur Auslassspitze in die Lungenarterie weiter transportiert. Ein solcher Einsatz eines Rechtsherzunterstützungssystems auf der Basis der erfindungsgemäßen Vorrichtung kann bei Patienten mit akutem Rechtsherzversagen zur Entlastung eingesetzt werden. Diese Patienten leiden oft an einer Organschädigung in Folge des Blutrückstaus, welcher durch einen verbesserten Abtransport aus dem rechten Vorhof und der Hohlvene mit diesem System behoben werden kann.

Die vorliegende Erfindung kombiniert die Vorteile der intraaortalen Ballonpumpe zur Nachlastsenkung und Verbesserung der Organperfusion mit dem Konzept von axialen Schraubenpumpen, welche bislang nur mit Mikroturbinen betrieben werden konnten.

Es wird eine Vorrichtung 1 zur Unterstützung des Herzens und des Kreislaufes für die Zirkulation von Körperflüssigkeiten angegeben, wobei der flüssigkeitsführende Hohlkörper der Vorrichtung 1 wenigstens eine von der Körperflüssigkeit durchströmbare Leitung bzw. Pumpenkanüle 2 mit mindestens einem Einlass und mindestens einem Auslass aufweist. Mit dem Ziel, die Körperflüssigkeiten gerichtet innerhalb des Körpers zu transportieren, sind erfindungsgemäß der Pumpenkatheter 2 und die Pumpenkammer 5 mit Ventilen versehen. Über einen externen Antrieb 9 wird in der Pumpkammer die Körperflüssigkeit durch eine intermittierend mit Fluidum gefüllte Pumpenkammer 5 angesogen bzw. herausgepresst. Die Druckamplitude, Pumpengeschwindigkeit, das Zeitverhältnis zwischen Füllen und Entleeren sind dabei wählbar und den Erfordernissen anzupassen. Eine Triggerung kann dabei entsprechend den Drucken in den Körperhöhlen, den elektrischen Impulsen oder von Flusssignalen her erfolgen. Es ist jedoch auch eine unabhängige (interne) Pumpaktion mit frei gewählter Frequenz, Amplitude und Phase möglich. Eine Einbringung des Systems ist mittels Röntgendurchleuchten, Ultraschall oder auch ohne Bildgebung mittels Führungskatheter und/oder Führungsdraht nach Gefäßpunktion perkutan möglich.

Das Einbringen des vorgestellten Kreislaufunterstützungssystems kann durch die Haut erfolgen. Eine Lagekontrolle bei der Einbringung des Systems ist mittels Röntgendurchleuchten, Ultraschall oder auch ohne Bildgebung (z.B. während Reanimation) mittels Führungskatheter und/oder Führungsdraht nach Gefäßpunktion perkutan möglich. Druckmessung an der Katheterspitze können Hinweise für die korrekte Lage des Pumpenkatheters 1 geben. Durch eine Bauart bestimmte Stabilisierung der äußeren Pumpenkammer 7 mit einer Mehrzahl von faltbaren Stegen oder einer entsprechenden Ausformung der äußeren Hülle, kann durch eine externe Manipulation die äußere Pumpenkammer 7 in einem mechanisch stabilen Zustand aufgestellt und eingebracht werden. Dabei ist die Größe und Struktur dieser äußeren Pumpenkammer 7 so gestaltet, dass sie nicht zu einer vollständigen Verlegung des großen Gefäßes (Aorta, vena cava) führt. Der innere Teil 6 der Pumpenkammer kann entweder gleichzeitig mit dem äußeren Teil 7 der Pumpenkammer eingebracht werden oder durch ein entsprechend vorgesehenes Innenlumen später eingeführt werden. Zum Entfernen des Kathetersystems bzw. der Vorrichtung 1 wird entsprechend in umgekehrter Reihenfolge die innere Kammer 6 evakuiert und gegebenenfalls wieder entfernt. Dann wird die äußere Pumpenkammer 7 wieder zusammengefaltet und kann über den perkutanen Zugang nach außen heraus gezogen werden.

Die Vorteile der vorgestellten erfindungsgemäßen Lösung liegen in der minimal invasiven Einbringung und Entfernung des Systems durch die Zusammenfaltbarkeit, die Einfachheit des Pumpenantriebes ohne rotierende Fremdkörper, welche zu einer verstärkten Zerstörung von Blutzellen (Hämolyse) führen können, der Kombination einer Entlastung des Herzens mit gleichzeitiger Unterstützung des Kreislaufs, sowie der Einfachheit eines universell ersetzbaren Antriebes. Solche Antriebskonsolen, welche für intraaortale Ballonpumpen (IABP) konzipiert sind, stehen in zahlreichen Kliniken zur Verfügung. Diese Antriebskonsolen können auch für die vorliegende Vorrichtung zum Betrieb verwendet werden, weshalb zusätzliche technische Apparaturen hier nicht gesondert bereit gestellt werden müssen. Folglich stellt dieses System nicht nur eine medizinisch, sondern auch ökonomisch sinnvolle Ergänzung derzeitig verfügbarer Kreislaufunterstützungssysteme dar. Die Einsatzmöglichkeiten am linken und am rechten Herzen sowie unter Verwendung von zwei externen Antrieben auch an beiden Herzkammern gleichzeitig stellen eine weitere medizinische Verbesserung für die Behandlung von Patienten mit akuter Herzschwäche dar.

Somit schafft die vorliegende Erfindung eine Vorrichtung zur Unterstützung des Herzens und des Kreislaufs für die Zirkulation von Körperflüssigkeiten, wobei der flüssigkeitsführende Hohlkörper wenigstens eine von der Körperflüssigkeit durchströmbare Leitung mit mindestens einem Einlass und mindestens einem Auslass aufweist, wobei ein Transport von Körperflüssigkeiten in gerichteter Weise innerhalb des Körpers stattfindet. Dabei ist der Pumpenkatheter und die Pumpenkammer mit Ventilen versehen. Die Körperflüssigkeit wird über einen pneumatischen Antrieb, welcher sich extern befindet oder in miniaturisierter Weise in den Körper implantiert wird, in der Pumpenkammer durch eine intermittierend mit Fluidium gefüllte Pumpenkammer angesogen bzw. herausgepresst. Die Druckamplitude, die Pumpgeschwindigkeit, das Zeitverhältnis zwischen Füllen und Entleeren sind dabei frei wählbar und den Erfordernissen anzupassen. Die Triggerung erfolgt entsprechend den Drucken in den Körperhöhlen, den elektrischen Impulsen oder von Flusssignalen, oder eine unabhängige (interne) Pumpaktion mit frei gewählter Frequenz, Amplitude und Phase ist möglich. Der Pumpenkatheter kann zudem eine faltbare und damit expanierbare Pumpenkammer aufweisen, welche zum Entfernen wieder zusammengefaltet werden kann. Der Pumpenkatheter kann zudem ohne chirurgische Maßnahmen, d.h. nur mittels Punktion durch die Haut über eine periphere Vene (rechtes Herz) oder Arterie (linkes Herz) eingebracht und vorgeschoben werden. Der Pumpenkatheter kann unter örtlicher Betäubung vorteilhaft und auch bei Reanimation und Kreislaufstillstand eingebracht und später wieder entfernt werden. Ein Betrieb des Pumpenkatheters mit einer handelsüblichen Antriebseinheit einer intraaortalen Ballonpumpe (IABP) kann bewerkstelligt werden. Das Pumpverfahren kann je nach Ausführung zur Unterstützung der rechten oder der linken Herzkammer oder für beide Herzkammern (2-Pumpkatheter) eingesetzt werden. Der vorgestellte Pumpenkatheter kann über eine IABP-Einheit auch bei Patienten betrieben werden, die eine höhergradige Aortenklappeninsuffizienz (eigentlich Kontraindikation für IABP) aufweisen.

Zum Betrieb des Pumpsystems als Pumpkammer können handelsübliche IABP-Ballonkatheter über ein Abdichten des Ventils eingebracht und betrieben werden. Der gesamte Pumpemkatheter besteht vorzugsweise aus biokompatiblen Materialien, d.h. es geht keine Internation oder Schädigung des Organismus von der Vorrichtung aus. Schließlich kann die Implantation über einen Katheter oder einen zuvor eingebrachten Führungsdraht, für welchen ein Innenlumen vorgesehen ist, erfolgen.

Obwohl die vorliegende Erfindung anhand bevorzugter Ausführungsbeispiele vorstehend beschrieben wurde, ist sie darauf nicht beschränkt, sondern auf vielfältige Weise modifizierbar.

### Bezugszeichenliste

- 1: Vorrichtung
- 2: Pumpenkanüle
- 3: Kanülenende mit Einlassöffnung
- 4: Einlassventil
- 5: Pumpeneinrichtung
- 6: innere Kammer
- 7: äußere Kammer
- 8: Auslassventil
- 9: Antriebseinrichtung
- 10: Körper
- 11: linke Herzkammer/linker Ventrikel
- 12: Auslassventil
- 13: Auslassventil
- 14: rechte Herzkammer/rechter Ventrikel
- 15: Auslassöffnung
- 16: Einlassventil
- 18: A. pulmonalis
- 19: rechter Vorhof
- 20: V. cava superior
- 21: V. cava inferior

## Patentansprüche

1. Vorrichtung (1) zur Zirkulation einer Körperflüssigkeit in einem Körper eines Lebewesens, insbesondere zum Unterstützen des Herzens und/oder des Kreislaufes des Lebewesens, mit:
einer Kathetereinrichtung, welche mindestens einen Einlassabschnitt (3; 16) zur Aufnahme der Körperflüssigkeit an mindestens einem ersten Ort innerhalb des Körpers des Lebewesens, mindestens einen von dem mindestens einen Einlassabschnitt (3; 16) beabstandeten Auslassabschnitt (8, 12, 13; 15) zur Abgabe der Körperflüssigkeit an mindestens einem von dem mindestens einen ersten Ort beabstandeten zweiten Ort innerhalb des Körpers des Lebewesens und eine Pumpeneinrichtung (5) für einen gerichteten Transport der Körperflüssigkeit zwischen dem mindestens einen Einlassabschnitt (3; 16) und dem mindestens einen Auslassabschnitt (8, 12, 13; 15) der Kathetereinrichtung aufweist, wobei die Pumpeneinrichtung (5) aus einem zusammenfaltbaren Doppelkammer-Hohlkörper mit einer inneren Kammer (6) und einer äußeren Kammer (7) zum Einbringen und Vorschieben der Vorrichtung (1) lediglich mittels Punktion durch die Haut über eine periphere Vene oder Arterie des Lebewesens besteht; und
einer mit der Kathetereinrichtung gekoppelten Ventilanordnung (4, 8, 12, 13; 16) für eine in Abhängigkeit des Betriebes der Pumpeneinrichtung (5) gesteuerte Aufnahme der Körperflüssigkeit an dem mindestens einen Einlassabschnitt (3; 16) der Kathetereinrichtung und gesteuerte Abgabe der Körperflüssigkeit an dem mindestens einen Auslassabschnitt (8, 12, 13; 15) der Kathetereinrichtung.

2. Vorrichtung nach Anspruch 1, **dadurch gekennzeichnet, dass**
die Kathetereinrichtung ein von der Pumpeneinrichtung (5) beabstandetes Kanülenende (3) und eine Pumpenkanüle (2) aufweist, welche als Transportleitung zwischen dem Kanülenende (3) und der Pumpeneinrichtung (5) angeordnet ist.

3. Vorrichtung nach Anspruch 2, **dadurch gekennzeichnet, dass**
die Ventilanordnung mindestens ein Einlassventil (4) aufweist, welches in der Pumpenkanüle (2) benachbart zu dem Einlassabschnitt (3; 16) angeordnet ist.

4. Vorrichtung nach wenigstens einem der vorhergehenden Ansprüche, **dadurch gekennzeichnet, dass**
die Pumpeneinrichtung (5) eine mit der Pumpenkammer koppelbare Antriebseinrichtung (9), insbesondere einen pneumatischen Antrieb, aufweist.

5. Vorrichtung nach Anspruch 2, **dadurch gekennzeichnet, dass**
das Kanülenende (3) als Ansaugkanüle ausgebildet ist und den mindestens einen Einlassabschnitt (3) der Kathetereinrichtung bildet.

6. Vorrichtung nach wenigstens einem der vorhergehenden Ansprüche, **dadurch gekennzeichnet, dass**
die Ventilanordnung mindestens ein Auslassventil (8, 12) im Bereich des Auslassabschnitts aufweist, wobei das mindestens eine Auslassventil (8, 12) an der äußeren Wandung der äußeren Kammer (7) der Pumpeneinrichtung (5) angeordnet ist.

7. Vorrichtung nach Anspruch 2, **dadurch gekennzeichnet, dass**
das Kanülenende (3) mindestens einen Auslassabschnitt (15) der Kathetereinrichtung bildet.

8. Vorrichtung nach wenigstens einem der Ansprüche 1 bis 4 und 7, **dadurch gekennzeichnet, dass**
der mindestens eine Einlassabschnitt der Kathetereinrichtung im Bereich der Pumpeneinrichtung (5) angeordnet ist.

9. Vorrichtung nach wenigstens einem der Ansprüche 1 bis 4 und 7 bis 8, **dadurch gekennzeichnet, dass**
die Ventilanordnung mindestens ein Einlassventil (16) im Bereich eines Einlassabschnitts (5) aufweist, wobei das mindestens eine Einlassventil (16) an der äußeren Wandung der äußeren Kammer (7) oder angrenzenden Abschnitten der Pumpeneinrichtung (5) ausgebildet ist.

10. Vorrichtung nach wenigstens einem der vorhergehenden Ansprüche, **dadurch gekennzeichnet, dass**
die Ventilanordnung mindestens ein Auslassventil (13) aufweist, wobei das mindestens eine Auslassventil (13) an der Wandung der Pumpenkanüle (2) der Kathetereinrichtung angeordnet ist.

11. Vorrichtung nach wenigstens einem der vorhergehenden Ansprüche, **dadurch gekennzeichnet, dass**
die Vorrichtung (1) aus biokompatiblen Materialien für eine Einführung in den Körper des Lebewesens ohne Interaktion oder Schädigung des Organismus besteht.

## Claims

1. A device (1) for circulating a body fluid in a body of a living organism, especially to support the heart and/or circulation of the living organism, comprising:
- a catheter device which has at least one inlet portion (3; 16) to take up the body fluid at at least one first location within the body of the living organism, at least one outlet portion (8, 12, 13; 15) some distance from the at least one inlet portion (3; 16) to discharge the body fluid at at least one second location some distance from the at least one first location within the body of the living organism and a pump device (5) for directed transport of the body fluid between the at least one inlet portion (3; 16) and the at least one outlet portion (8, 12, 13; 15) of the catheter device, the pump device (5) comprising a collapsible double chamber cavity with an inner chamber (6) and an outer chamber (7) for inserting and feeding the device (1) solely by means of puncturing the skin via a peripheral vein or artery in the living organism; and
- a valve arrangement (4, 8, 12, 13; 16) coupled to the catheter device for controlled uptake of the body fluid as a function of operation of the pump device (5) at the at least one inlet portion (3; 16) of the catheter device and controlled discharge of the body fluid at the at least one outlet portion (8, 12, 13; 15) of the catheter device.

2. A device according to claim 1, **characterised in that** the catheter device has a cannula end (3) at some distance from the pump device (5) and a pump cannula (2) which is arranged as a transport line between the cannula end (3) and the pump device (5).

3. A device according to claim 2, **characterised in that** the valve arrangement has at least one inlet valve (4) which is located in the pump cannula (2) adjacent to the inlet portion (3; 16) .

4. A device according to at least one of the preceding claims, **characterised in that** the pump device (5) has a drive mechanism (9), in particular a pneumatic drive, which may be coupled to the pump chamber.

5. A device according to claim 2, **characterised in that** the cannula end (3) is designed as a suction cannula and forms the at least one inlet portion (3) of the catheter device.

6. A device according to at least one of the preceding claims, **characterised in that** the valve arrangement has at least one outlet valve (8, 12) in the region of the outlet portion, the at least one outlet valve (8, 12) being located on the outer wall of the outer chamber (7) of the pump device (5).

7. A device according to claim 2, **characterised in that** the cannula end (3) forms at least one outlet portion (15) of the catheter device.

8. A device according to at least one of claims 1 to 4 and 7, **characterised in that** the at least one inlet portion of the catheter device is located in the region of the pump device (5).

9. A device according to at least one of claims 1 to 4 and 7 to 8, **characterised in that** the valve arrangement has at least one inlet valve (16) in the region of an inlet portion (5), the at least one inlet valve (16) being provided on the outer wall of the outer chamber (7) or adjacent portions of the pump device (5).

10. A device according to at least one of the preceding claims, **characterised in that** the valve arrangement has at least one outlet valve (13), the at least one outlet valve (13) being located on the wall of the pump cannula (2) of the catheter device.

11. A device according to at least one of the preceding claims, **characterised in that** the device (1) is made from biocompatible materials for insertion into the body of the living organism without interacting with or damaging the organism.

## Revendications

1. Dispositif (1) permettant la circulation d'un liquide corporel dans le corps d'un être vivant, en particulier l'assistance du coeur et/ou du système circulatoire de l'être vivant, comprenant :
- un dispositif de cathéter, qui présente au moins une section d'admission (3 ; 16) permettant de réceptionner le liquide corporel à au moins un premier endroit dans le corps de l'être vivant, au moins une section d'évacuation (8, 12, 13 ; 15) distante de la au moins une section d'admission (3 ; 16) et permettant d'évacuer le liquide corporel à au moins un deuxième endroit distant du au moins un premier endroit dans le corps de l'être vivant, et un dispositif de pompe (5) pour un transport orienté du liquide corporel entre la au moins une section d'admission (3 ; 16) et la au moins une section d'évacuation (8, 12, 13 ; 15) du dispositif de cathéter, le dispositif de pompe (5) étant constitué d'un corps creux à double chambre repliable doté d'une chambre intérieure (6) et d'une chambre extérieure (7) permettant d'insérer et d'avancer le dispositif (1) simplement par ponction à travers la peau via une veine ou une artère périphérique de l'être vivant ; et
- un ensemble de valves (4, 8, 12, 13 ; 16) accouplé au dispositif de cathéter pour une réception du liquide corporel, régulée en fonction du fonctionnement du dispositif de pompe (5), à la au moins une section d'admission (3 ; 16) du dispositif de cathéter et une évacuation régulée du liquide corporel à la au moins une section d'évacuation (8, 12, 13 ; 15) du dispositif de cathéter.

2. Dispositif selon la revendication 1, **caractérisé en ce que** le dispositif de cathéter présente une extrémité de canule (3) distante du dispositif de pompe (5) et une canule de pompe (2) qui est disposée entre l'extrémité de canule (3) et le dispositif de pompe (5) en tant que conduite de transport.

3. Dispositif selon la revendication 2, **caractérisé en ce que** l'ensemble de valves comprend au moins une valve d'admission (4), qui est disposée dans la canule de pompe (2) à proximité de la section d'admission (3 ; 16).

4. Dispositif selon au moins une des revendications précédentes, **caractérisé en ce que** le dispositif de pompe (5) présente un système d'entraînement (9), en particulier un entraînement pneumatique, pouvant être accouplé à la chambre de pompe.

5. Dispositif selon la revendication 2, **caractérisé en ce que** l'extrémité de canule (3) est conçue sous forme de canule d'aspiration et forme la au moins une section d'admission (3) du dispositif de cathéter.

6. Dispositif selon au moins une des revendications précédentes, **caractérisé en ce que** l'ensemble de valves comprend au moins une valve d'évacuation (8, 12) dans la zone de la section d'évacuation, la au moins une valve d'évacuation (8, 12) étant fixée à la paroi extérieure de la chambre extérieure (7) du dispositif de pompe (5).

7. Dispositif selon la revendication 2, **caractérisé en ce que** l'extrémité de canule (3) forme au moins une section d'évacuation (15) du dispositif de cathéter.

8. Dispositif selon au moins une des revendications 1 à 4 et 7, **caractérisé en ce que** la au moins une section d'admission du dispositif de cathéter est disposée dans la zone du dispositif de pompe.

9. Dispositif selon au moins une des revendications 1 à 4 et 7 à 8, **caractérisé en ce que** l'ensemble de valves comprend au moins une valve d'admission (16) dans la zone de la section d'admission (5), la au moins une valve d'admission (16) étant conçue sur la paroi extérieure de la chambre extérieure (7) ou sur des sections adjacentes du dispositif de pompe (5).

10. Dispositif selon au moins une des revendications précédentes, **caractérisé en ce que** l'ensemble de valves comprend au moins une valve d'évacuation (13), la au moins une valve d'évacuation (13) étant fixée à la paroi de la canule de pompe (2) du dispositif de cathéter.

11. Dispositif selon au moins une des revendications précédentes, **caractérisé en ce que** le dispositif (1) est constitué de matériaux biocompatibles pour une introduction dans le corps de l'être vivant sans interaction ou dégradation de l'organisme.
